# FASCICULE DE BREVET EUROPEEN

(11) **EP 2 627 778 B1**
(45) Date de publication et mention de la délivrance du brevet: **30.12.2015**
(21) Numéro de dépôt: 11787710.0
(22) Date de dépôt: 17.10.2011
(51) Int. Cl.: C12P 19/04, C12N 9/16

(54) **PROCEDE DE TRANSFORMATION DU IOTA-CARRAGHENANE EN ALPHA-CARRAGHENANE A L'AIDE D'UNE NOUVELLE CLASSE DE 4S-IOTA-CARRAGHENANE SULFATASE**
VERFAHREN ZUR UMWANDLUNG VON IOTA-CARRAGEEN IN ALPHA-CARRAGEEN ANHAND EINER NEUEN 4S-IOTA-CARRAGEEN-SULFATASEKLASSE
METHOD FOR TRANSFORMING IOTA-CARRAGEENAN INTO ALPHA-CARRAGEENAN BY MEANS OF A NEW CLASS OF 4S-IOTA-CARRAGEENAN SULFATASE

(30) Priorité: 15.10.2010 FR 1058420
(43) Date de publication de la demande: 21.08.2013
(73) Titulaire: Centre National de la Recherche Scientifique (CNRS), 75794 Paris Cedex 16 (FR)
(72) Inventeur: HELBERT, William, F-38410 SAINT-MARTIN-D'URIAGE (FR); PRECHOUX, Aurélie, F-29370 Elliant (FR); GENICOT-JONCOUR, Sabine, F-29250 Saint-pol-de-leon (FR)
(74) Mandataire: Novagraaf Technologies
(86) Numéro de dépôt international: PCT/FR2011/052421
(87) Numéro de publication internationale: WO 2012/049437

(56) Documents cités:
- WO-A2-00/68395
- FALSHAW R ET AL: "Structural analysis of carrageenans from Burmese and Thai samples of Catenella nipae Zanardini", CARBOHYDRATE RESEARCH, PERGAMON, GB, vol. 285, 14 mai 1996 (1996-05-14), pages 81-98, XP004018644, ISSN: 0008-6215, DOI: 10.1016/0008-6215(96)00031-6
- GENICOT-JONCOUR S ET AL: "The cyclization of the 3,6-anhydro-galactose ring of iota-carrageenan is catalyzed by two D-galactose-2,6-sulfurylases in the red alga Chondrus crispus", PLANT PHYSIOLOGY, AMERICAN SOCIETY OF PLANT PHYSIOLOGISTS, ROCKVILLE, MD, US, vol. 151, no. 3, 1 novembre 2009 (2009-11-01), pages 1609-1616, XP002598802, ISSN: 0032-0889, DOI: 10.1104/PP.109.144329 [extrait le 2009-09-04]
- DATABASE UniProt [Online] 25 juillet 2006 (2006-07-25), "SubName: Full=Sulfatase; Flags: Precursor;", XP002640183, extrait de EBI accession no. UNIPROT:Q15XH3 Database accession no. Q15XH3 cité dans la demande
- MCLEAN M W ET AL: "Glycosulphatase from Pseudomonas carrageenovora. Purification and some properties.", EUROPEAN JOURNAL OF BIOCHEMISTRY, BLACKWELL PUBLISHING, BERLIN, DE, vol. 101, no. 2, 1 novembre 1979 (1979-11-01), pages 497-505, XP000961727, ISSN: 0014-2956, DOI: 10.1111/J.1432-1033.1979.TB19744.X
- MICHEL GURVAN ET AL: "Bioconversion of red seaweed galactans: a focus on bacterial agarases and carrageenases", APPLIED MICROBIOLOGY AND BIOTECHNOLOGY, SPRINGER VERLAG, BERLIN, DE, vol. 71, no. 1, 1 juin 2006 (2006-06-01), pages 23-33, XP002562259, ISSN: 0175-7598, DOI: 10.1007/S00253-006-0377-7 [extrait le 2006-03-21]

### SEQUENCE LISTING

<110> Centre National de la Recherche Scientifique - CNRS PRECHOUX, Aurélie GENICOT-JONCOUR, Sabine HELBERT, WILLIAM
<120> Procédé de transformation du iota-carraghénane en alpha-carraghénane à l'aide d'une nouvelle classe de 4S-iota-carraghénane sulfatase
<130> BIP207246PC00
<150> FR1058420
   <151> 2010-10-15
<160> 6
<170> PatentIn version 3.5
<210> 1
   <211> 18
   <212> PRT
   <213> Pseudoalteromonas atlantica
<400> 1
   Asn Gly Gln Phe Asp Asn Thr Val Ile Val Phe Thr Ser Asp Asn Gly
   1               5                   10                  15
   Gly Lys
<210> 2
   <211> 12
   <212> PRT
   <213> Pseudoalteromonas atlantica
<400> 2
   Phe Asp Gln Thr Phe Gln Val Gly Asp Asn Thr Arg
   1               5                   10
<210> 3
   <211> 11
   <212> PRT
   <213> Pseudoalteromonas atlantica
<400> 3
   Glu Thr Glu Tyr Ile Thr Asp Gly Leu Ser Arg
   1 5 10
<210> 4
   <211> 500
   <212> PRT
   <213> Pseudoalteromonas atlantica
<400> 4
   Met Thr Phe Asn Lys Lys Val Ser Thr Leu Leu Trp Gly Thr Leu Ile
   1               5                   10                  15
   Ala Ile Ser Val Gly Asn Ala Ser Ala Ala Asp Ala Gly Gln Ser Lys
               20                  25                  30
   Ala Asp Glu Ser Asn Glu Lys Pro Asn Ile Leu Phe Val Leu Ala Asp
           35                  40                  45
   Asp Leu Gly Tyr Asn Asp Val Gly Phe Asn Gly Ser Thr Asp Ile Lys
       50                  55                  60
   Thr Pro Asn Leu Asp Gly Leu Ala Lys Asn Gly Met Thr Phe Asp Ala
   65                  70                  75                  80
   Ala Tyr Val Ala His Pro Phe Cys Gly Pro Ser Arg Ala Ala Ile Met
                   85                  90                  95
   Thr Gly Arg Tyr Pro His Lys Ile Gly Ala Gln Phe Asn Leu Pro Glu
               100                 105                 110
   Asp Asn Ser Asn Val Gly Val Ser Ala Asp Glu Leu Phe Ile Ala Gln
           115                 120                 125
   Thr Met Lys Ser Ala Gly Tyr Phe Thr Gly Ala Met Gly Lys Trp His
       130                 135                 140
   Leu Gly Glu Ala Ser Glu Tyr His Pro Asn Lys His Gly Phe Asp Glu
   145                 150                 155                 160
   Phe Tyr Gly Phe Leu Gly Gly Gly His Asn Tyr Phe Pro Glu Gln Phe
                   165                 170                 175
   Glu Ala Ala Tyr Asn Lys Arg Val Ala Gln Gly Met Thr Asn Ile Asn
               180                 185                 190
   Met Tyr Leu Thr Pro Leu Glu His Asn Gly Lys Glu Val Arg Glu Thr
           195                 200                 205
   Glu Tyr Ile Thr Asp Gly Leu Ser Arg Glu Ala Val Asn Phe Val Asp
       210                 215                 220
   Lys Ala Ala Ala Lys Lys Lys Pro Phe Phe Leu Tyr Leu Ala Tyr Asn
   225                 230                 235                 240
   Ala Pro His Val Pro Leu Gln Ala Lys Glu Glu Asp Met Ala Met Phe
                   245                 250                 255
   Ser Gln Ile Lys Asp Lys Lys Arg Arg Thr Tyr Ala Gly Met Val Tyr
               260                 265                 270
   Ala Val Asp Arg Gly Val Gly Arg Ile Val Glu Gln Leu Lys Lys Asn
           275                 280                 285
   Gly Gln Phe Asp Asn Thr Val Ile Val Phe Thr Ser Asp Asn Gly Gly
       290                 295                 300
   Lys Leu Gly Gln Gly Ala Asn Asn Tyr Pro Leu Lys Glu Gly Lys Gly
   305                 310                 315                 320
   Ser Val Gln Glu Gly Gly Phe Arg Thr Pro Met Leu Val His Trp Pro
                   325                 330                 335
   Lys His Met Lys Ala Gly Ser Arg Phe Ser His Pro Val Leu Ala Leu
               340                 345                 350
   Asp Leu Tyr Pro Thr Phe Ala Gly Leu Gly Gly Ala Val Leu Pro Glu
           355                 360                 365
   Asp Lys Lys Leu Asp Gly Lys Asp Ile Trp Ala Asp Ile Gln Ala Asn
       370                 375                 380
   Thr Ala Pro His Lys Asp Glu Phe Ile Tyr Val Leu Arg His Arg Asn
   385                 390                 395                 400
   Gly Tyr Ser Asp Ala Ala Ala Arg Arg Asn Gln Phe Lys Ala Val Lys
                   405                 410                 415
   Asn His Asn Asp Asp Trp Lys Leu Tyr Asn Ile Ala Gln Asp Ile Ser
               420                 425                 430
   Glu Asp Asn Asp Ile Ser Ala Gln His Pro Asp Ile Leu Arg Asp Met
           435                 440                 445
   Val Ser Ser Met Glu Ser Trp Ser Trp Asn Asn Gln Gln Pro Lys Trp
       450                 455                 460
   Phe His Gln Ser Ala Glu Gly Ala Gln Trp Arg Leu Lys Ala Met Pro
   465                 470                 475                 480
   Arg Phe Asp Gln Thr Phe Gln Val Gly Asp Asn Thr Arg Ser Asn Ser
                   485                 490                 495
   Lys Lys Gly His
               500
<210> 5
   <211> 11
   <212> PRT
   <213> Pseudoalteromonas atlantica
<220>
   <221> MISC_FEATURE
   <222> (1)..(1)
   <223> Xaa représente C ou S
<220>
   <221> MISC_FEATURE
   <222> (2)..(2)
   <223> Xaa représente n'importe quel acide aminé
<220>
   <221> MISC_FEATURE
   <222> (4)..(4)
   <223> Xaa représente n'importe quel acide aminé, de préférence S
<220>
   <221> MISC_FEATURE
   <222> (6)..(8)
   <223> Xaa représente n'importe quel acide aminé
<220>
   <221> MISC_FEATURE
   <222> (9)..(9)
   <223> Xaa représente n'importe quel acide aminé, de préférence L
<220>
   <221> MISC_FEATURE
   <222> (10)..(10)
   <223> Xaa représente n'importe quel acide aminé, de préférence G
<220>
   <221> MISC_FEATURE
   <222> (11)..(11)
   <223> Xaa représente n'importe quel acide aminé, de préférence R
<400> 5
   Xaa Xaa Pro Xaa Arg Xaa Xaa Xaa Xaa Xaa Xaa
   1               5                   10
<210> 6
   <211> 12
   <212> PRT
   <213> Pseudoalteromonas atlantica
<220>
   <221> MISC_FEATURE
   <222> (2)..(2)
   <223> Xaa représente Y ou V
<220>
   <221> MISC_FEATURE
   <222> (3)..(3)
   <223> Xaa représente n'importe quel acide aminé
<220>
   <221> MISC_FEATURE
   <222> (4)..(4)
   <223> Xaa représente S ou T
<220>
   <221> MISC_FEATURE
   <222> (5)..(7)
   <223> Xaa représente n'importe quel acide aminé
<220>
   <221> MISC_FEATURE
   <222> (10)..(11)
   <223> Xaa représente n'importe quel acide aminé
<400> 6
   Gly Xaa Xaa Xaa Xaa Xaa Xaa Gly Lys Xaa Xaa His
   1               5                   10

## Description

### Domaine technique

La présente invention se rapporte à un procédé de transformation du iota-carraghénane en alpha-carraghéhane à l'aide d'une nouvelle classe de 4S-iota-carraghénane sulfatase.

La présente invention trouve notamment une application en industrie agro-alimentaire, pharmacie et cosmétique.

Dans la description ci-dessous, les références entre crochets ([ ]) renvoient à la liste des références présentée à la fin du texte.

### Etat de la technique

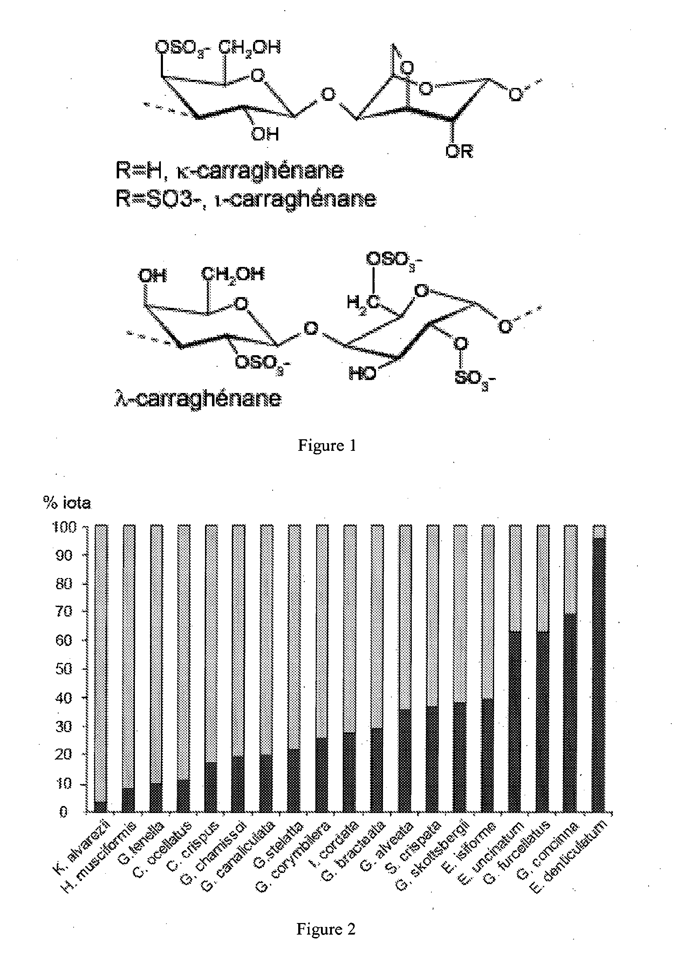

Les carraghénanes sont des galactanes sulfatés extraits de la paroi des algues rouges marines. Les carraghénanes sont composés d'un enchaînement de D-galactosides liés alternativement par des liaisons alpha(1-3) et bêta(1-4). Ces polysaccharides anioniques se distinguent essentiellement par la présence ou non d'un pont 3,6 anhydro sur le résidu galactose lié en alpha(1-3), et par leur taux de sulfatation. Par exemple les trois unités de répétition disacharidique - appelées motif carrabiose - rencontrées dans les carraghénanes les plus exploités industriellement sont caractérisées par la présence de un (kappa-carrabiose), deux (iota-carrabiose) ou trois sulfates (lambda-carrabiose) (figure 1). Les carraghénanes peuvent être composés principalement d'un motif carrabiose, par exemple le kappa-carraghénane de l'algue *Kappaphycus alvarezzi* est composé d'environ 90% de motif kappa-carrabiose et 10% de iota-carrabiose. Le iota-carraghénane extrait d'*Eucheuma denticulatum* est composé de 85% d'unités iota-carrabiose et 15% d'unités kappa-carrabiose.

Les compositions en unité carrabiose peuvent être très variables et dépendent essentiellement de l'origine botanique de l'algue. On parle de kappa-carraghénane quand le polysaccharide est riche en motif kappa-carrabiose et que ces propriétés physico-chimiques sont proches de celles du kappa-carraghénane de *K. alvarezzi* souvent utilisé comme référence. (Bixler et al., Food Hydocolloids, 15 : 619-630, 2001) [1].

Toute une gamme de structures intermédiaires de kappa/iota-carraghénanes hybrides a été décrite en fonction de l'origine botanique des polysaccharides (figure 2 ; Bixler et al., 2001, précité) [1]. Le type de carraghénane présent dans la paroi des algues peut également être corrélé au stade de vie des algues. En effet dans le cas de *Chondrus crispus*, les gamétophytes sont riches en kappa/iota- carraghénanes tandis que les sporophytes contiennent essentiellement du lambda-carraghénane. Les saisons et tous les facteurs environnementaux pouvant affecter la croissance des algues (éclairement, température, sels, etc...) auront également un effet sur la structure et la composition en carraghénane. Par conséquent, en fonction de l'origine et/ou des procédures d'extraction, un large spectre de structures de type kappa-, iota- et lambda-carraghénanes peut être observé.

Ces polysaccharides ont des propriétés rhéologiques uniques et sont utilisés comme agents de texture en industrie agro-alimentaire, pharmacie et cosmétique. Ces polysaccharides possèdent un large spectre de propriétés fonctionnelles qui s'explique par leur grande diversité structurale. Les kappa- et iota-carraghénanes ont la propriété de former des gels iono- et thermo-dépendants. Le kappa-carraghénane va former des gels rigides en présence de potassium alors que le iota-carraghénane forme des gels souples et élastiques en présence de calcium. La grande diversité de structure chimique des carraghénanes et leur hybridicité naturelle confèrent à chaque extrait d'algue des propriétés fonctionnelles caractéristiques.

Environ 50 000 tonnes de carraghénanes sont vendus annuellement (Bixler and Porse, J. Appl. Phycol., 2010, online) [2]. Toutefois, le tonnage des carraghénanes exploités est limité par la quantité d'algue rouge disponible. Actuellement, deux espèces d'algue rouge sont largement cultivées : *Kappaphycus alvarezzi* et *Eucheuma denticulatum* dont sont extraits le kappa- et le iota-carraghénane, respectivement. De nombreuses algues sauvages (non cultivées) sont également collectées en grande quantité car leurs carraghénanes, de nature kappa/iota-hybrides, présentent des propriétés fonctionnelles très intéressantes. Cependant la tonne de ces algues est deux fois voire dix fois plus chère que celle des algues cultivées.

En outre, à chaque application industrielle correspondent des extraits de carraghénanes provenant d'une espèce ou de plusieurs espèces d'algues rouges. Les solutions pour satisfaire les besoins industriels dans n'importe quel domaine résident essentiellement dans la formulation (le mélange) des carraghénanes (Bixler and Porse, 2010, précité) [2].

Par conséquent, un procédé biotechnologique qui permettrait d'obtenir des carraghénanes hybrides à partir des algues cultivées serait économiquement très rentable. Il présenterait l'avantage d'être moins dépendant de la source d'algues, et ouvrirait de nouvelles perspectives quant à l'exploitation et la valorisation de la biomasse d'algues rouges.

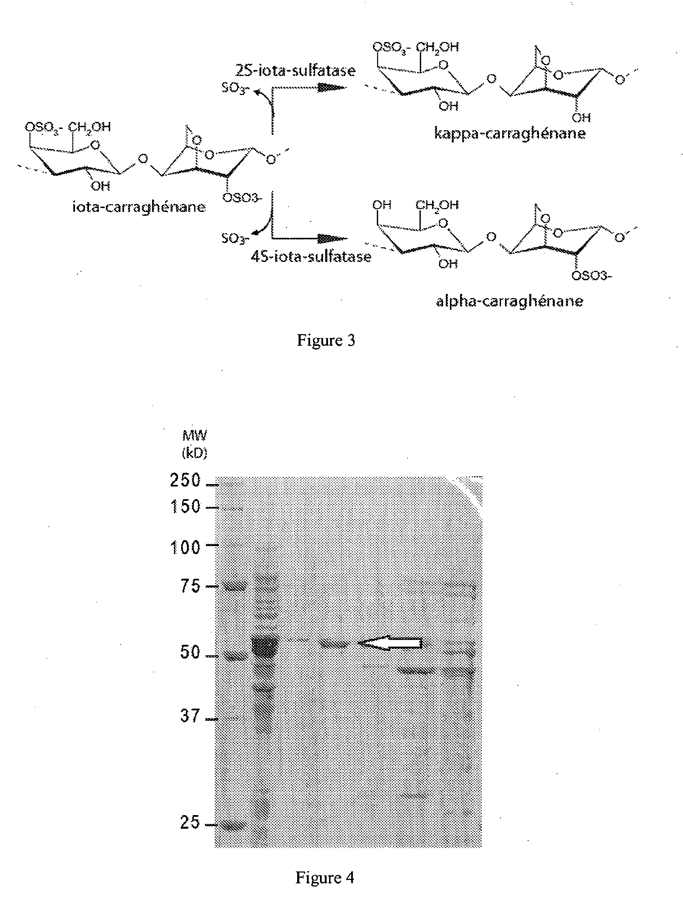

Afin de contrôler la structure chimique et, par extension, les propriétés physico-chimiques des carraghénanes, les Inventeurs ont donc entrepris la purification et la production d'enzymes capables de modifier et de corriger les structures des carraghénanes. Les modifications recherchées consistent en la désulfatation des carraghénanes par des enzymes appelées sulfatases qui conduisent en la conversion du iota- en kappa- ou alpha-carraghénane, ou à des structures hybrides de type kappa/iota- ou iota/alpha-carraghénane (figure 3). Ils ont ainsi démontré l'existence de carraghénane-sulfatases pouvant agir directement sur le polymère sans action préalable de carraghénases. Ils ont réussi à purifier à partir d'une population bactérienne *Pseudoalteromonas carrageenovora,* une première 4S-iota-carraghénane sulfatase appartenant à la famille des amidohydrolases, et qui convertit le iota- en alpha-carraghénane par désulfatation (élimination d'un groupe SO₃⁻) spécifique en position 4 du iota-carraghénane (Demande de Brevet français FR 09/52642 publiée sous le numéro FR 2944804) [3]. En utilisant la même stratégie que pour la première 4S-iota-carraghénane sulfatase, les Inventeurs ont réussi à purifier à partir de *Pseudoalteromonas atlantica,* une seconde 4S-iota-carraghénane sulfatase appartenant à la famille des formylglycine-dependent sulfatases. Ils ont tout d'abord réussi à déterminer trois peptides de ladite protéine : NGQFDNTVIVFTSDNGGK (SEQ ID NO 1), FDQTFQVGDNTR (SEQ ID NO 2), et ETEYITDGLSR (SEQ ID NO 3), qui une fois confrontés à la banque TrEMBL, ont mis en évidence une correspondance avec la protéine Q15XH3 de *P. atlantica* T6c dont le gène (PatI_0889) a été annoté comme une sulfatase (Protein_ID ABG39415.1 ; Copeland et al., 2006) [4].

Les 4S-iota-carraghénane sulfatases pourraient donc permettre de calibrer « l'hybridicité » des carraghénanes. Ainsi, toute sulfatase agissant sur le iota-carraghénane pourrait représenter une innovation majeure car elle permettrait de fabriquer des alpha- et iota/alpha- carraghénanes qui sont très peu abondants dans la nature, et différents en terme de séquence peptidique mais également de propriétés biochimiques. Toutefois il est apparu que les sulfatases agissant sur les carraghénanes pouvaient ne pas présenter d'homologie avec les autres sulfatases connues - les sulfatases les plus étudiées étant les enzymes agissant sur l'héparine, un polysaccharide d'origine animal.

Il existe donc un réel besoin de purifier des enzymes de modification des motifs de sulfatation des carraghénanes afin de pallier aux défauts, inconvénients et obstacles de l'art antérieur, en particulier d'un procédé permettant de maitriser « l'hybridicité » des carraghénanes utilisant lesdites enzymes, de réduire les coûts et de contrôler l'approvisionnement et les propriétés fonctionnelles des carraghénanes ainsi obtenus.

### Description de l'invention

Les Inventeurs ont mis en évidence de manière tout à fait inattendue que la seconde 4S-iota-carraghénane sulfatase identifiée (Q15XH3) est capable de convertir le motif iota-carrabiose en motif alpha-carrabiose. Le motif iota-carrabiose peu être présent dans un oligo-saccharide ou un polysaccharide qui peut être composé également d'autres motifs carrabiose.

La présente invention a donc pour objet un procédé de transformation du iota-carraghénane en alpha-carraghénane comprenant la catalyse enzymatique de la conversion du motif iota-carrabiose en motif alpha-carrabiose par une enzyme ayant une activité sulfatase et dont la séquence peptidique comprend les peptides de séquences suivantes :
NGQFDNTVIVFTSDNGGK (SEQ ID NO 1),
FDQTFQVGDNTR (SEQ ID NO 2), et
ETEYITDGLSR (SEQ ID NO 3).

On entend par « iota- et alpha-carraghénane » au sens de la présente invention, des iota- et alpha-carraghénanes et/ou iota- et alpha-carraghénanes hybrides, par exemple contenus en solution, solution partiellement gélifiée ou gel. Ainsi, le iota-carraghénane pur gélifie sous ses propres charges, alors que des carraghénanes hybrides tels que le iota-nu-carraghénane et les oligo-iota-carraghénanes sont peu gélifiants.

Selon un mode de réalisation particulier du procédé de l'invention, ladite enzyme a la séquence peptidique suivante :

Selon un mode de réalisation particulier du procédé de l'invention, ladite enzyme est une 4S-iota-carraghénane sulfatase. Par exemple, il peut s'agir d'une 4S-iota-carraghénane sulfatase de séquence présentant au moins 30% d'identité de séquence avec SEQ ID NO : 4, de préférence au moins 50% d'identité de séquence avec SEQ ID NO: 4, tout préférentiellement au moins 80% avec SEQ ID NO : 4.

Selon un mode de réalisation particulier du procédé de l'invention, l'enzyme est produite par une cellule hôte comprenant un acide nucléique codant pour ladite enzyme et/ou un vecteur comprenant une séquence d'acide nucléique codant pour ladite enzyme.

On entend par « cellule hôte » au sens de la présente invention une cellule procaryote ou eucaryote. Des cellules hôtes couramment utilisées pour l'expression de cellules recombinantes incluent notamment des cellules de bactéries telles que *Escherichia coli* ou *Bacillus,* des cellules de levures telles que *Saccharomyces cerevisiae*, des cellules de champignons tels que *Aspergillus niger*, des cellules d'insectes, et des cellules de mammifères (notamment humaine) telles que les lignées cellulaires CHO, HEK 293, PER-C6, etc... La transformation des cellules procaryotes et eucaryotes est une technique bien connue de l'homme du métier, par exemple la lipofection, l'électroporation, le choc thermique, ou des méthodes chimiques. En fonction de la cellule à transformer, l'homme du métier pourra aisément déterminer les moyens nécessaires à l'introduction et à l'expression de l'acide nucléique chez la cellule hôte choisie. Ainsi le vecteur d'expression et la méthode d'introduction du vecteur d'expression au sein de la cellule hôte seront sélectionnés en fonction de la cellule hôte choisie. La cellule hôte transformée par un vecteur d'expression ou un acide nucléique va exprimer le polypeptide correspondant de manière stable. L'homme du métier peut aisément vérifier que la cellule hôte exprime le polypeptide de manière stable, par exemple en utilisant la technique du Western blot.

On entend par « vecteur » au sens de la présente invention, des vecteurs d'expression et/ou de sécrétion de séquences d'acide nucléique dans une cellule hôte déterminée. Ils peuvent par exemple être des vecteurs d'origine plasmidique ou virale, comportant, outre la séquence d'acide nucléique, les moyens nécessaires à son expression. Ces moyens peuvent par exemple inclure un promoteur, des signaux d'initiation et de terminaison de la traduction, ainsi que des régions appropriées de régulation de la transcription. Le vecteur d'expression peut également comprendre d'autres éléments tels qu'une origine de réplication, un site de clonage multiple, un enhanceur, un peptide signal qui pourra être fusionné en phase avec le polypeptide produit lors du clonage, et un ou plusieurs marqueurs de sélection.

On entend par « acide nucléique » au sens de la présente invention, aussi bien des molécules d'ADN que des molécules d'ARN, ce qui inclut notamment des molécules d'ADNc et des molécules d'ARNm. L'acide nucléique peut être sous forme double brin (par exemple dans le cas d'un acide nucléique compris dans un vecteur d'expression) ou sous forme simple brin (par exemple dans le cas de sondes ou d'amorces).

Selon un mode de réalisation particulier du procédé de l'invention, les carraghénanes obtenus possèdent des motifs alpha-carrabioses dès lors que le carraghénane de départ possédait dans sa structure des motifs iota-carrabioses. Par exemple, les motifs iota-carrabioses du carraghénane extrait d'*E*. *denticulatum* (85% iota-carrabiose et 15% kappa-carrabiose) peuvent être convertis en un carraghénane possédant 85% d'alpha-carrabiose et 15% de kappa-carrabiose. La réaction enzymatique peut être contrôlée et le taux de conversion de iota-carrabiose en alpha-carrabiose peut varier de 0 à 100%. Les propriétés rhéologiques des carraghénanes obtenus seront modulées en fonction du taux de conversion.

D'autres avantages pourront encore apparaître à l'homme du métier à la lecture des exemples ci-dessous, illustrés par les figures annexées, donnés à titre illustratif.

### Brève description des figures

- La figure 1 représente la structure chimique des motifs de répétition des trois principaux carraghénanes (kappa κ, iota τ, et lambda λ) exploités industriellement.
- La figure 2 représente les compositions hybrides kappa/iota-carraghénanes en fonction de la source d'algues rouges (Bixler et al., 2001, précité) [1].
- La figure 3 représente le schéma des réactions enzymatiques catalysées par les 4S- et 2S-iota-carraghénane sulfatases.
- La figure 4 représente le gel d'électrophorèse (SDS-PAGE) coloré au bleu de Coomassie colloïdal de plusieurs fractions collectées après l'étape de chromatographie échangeuse d'anions (Q Sepharose Fast Flow). La protéine de 55 kDa est indiquée à l'aide d'une flèche.
- La figure 5 représente la séquence en acides aminés de la protéine de *Pseudoalteromonas atlantica* T6c (Q15XH3). En gras, les trois séquences peptidiques déterminées par spectrométrie de masse. En italique souligné, la signature de la modification post-traductionnelle de la cystéine. En encadré, les acides aminés du site catalytique dont la lysine (K) et l'histidine (H) qui sont les acides aminés catalytiques.
- La figure 6 représente l'environnement génomique du gène de la nouvelle 4S-iota-carraghénane sulfatase (Q15XH3) dans *P. atlantica*.
- La figure 7 représente le spectre ¹H RMN du iota-carraghénane (B) incubé avec la protéine purifiée de *Pseudoalteromonas atlantica* (Q15XH3) (A)

### EXEMPLES

### Exemple 1 : Identification d'une nouvelle 4S-iota-carraghénane sulfatase issue de Pseudoalteromonas atlantica

### Criblage des activités carraghénane-sulfatases de Pseudoalteromonas atlantica

Le criblage a été réalisé en utilisant la bactérie marine *Pseudoalteromonas atlantica* T6c (souche ATCC T6c / BAA-1087) dont le génome a été complètement séquencé (http://genome.igi-psf.org/finished microbes/pseat/pseat.home.html).

### Cultures bactériennes

La production des extraits bactériens a été réalisée à partir de la bactérie marine *Pseudoalteromonas atlantica* cultivée en milieu Zobell [Bacto peptone (*Amresco*) 5g/l, extraits de levure (BD, *Extract of Autolysed Yeast cells*) 1g/l, eau de mer filtrée 800ml complétée à 1l par de l'eau déminéralisée] en présence de kappa- (*Kappaphycus alvarezii*, CP Kelco X6913, à 1g/l), de iota- (*Eucheuma denticulatum,* H030058-534, à 1g/l) ou de lambda-carraghénane (sporophyte de *Gigartina skottsbergii,* CP Kelco, X7055).

La première préculture a consisté à ensemencer 10ml de milieu Zobell à partir d'un glycérol de *P. atlantica* conservé à -80°C. L'incubation a été réalisée dans un agitateur de type New Brunswick pendant 36h, à 18°C et 180rpm. Pour la deuxième préculture, 50ml de milieu Zobell contenant 50mg d'un des carraghénanes (kappa-, iota- ou lambda-) ont été ensemencés avec environ 1ml de la première préculture, de manière à avoir une absorbance de 0,1, à 660nm. Cette deuxième préculture a été incubée à 18°C jusqu'à ce que la densité optique atteigne une valeur de 1 à 1,2, à 660nm, soit environ 8h. Pour la culture finale, 950ml de milieu Zobell contenant le même carraghénane que précédemment, ont été ensemencés avec les 50ml de la deuxième préculture, et incubés à 18°C pendant 36h.

Afin de séparer le culot bactérien du surnageant de culture, les cultures bactériennes ont été centrifugées à 6200g pendant 20 minutes, à 4°C.

### Préparation des extraits bactériens

Le surnageant de culture est concentré par précipitation à 90% de saturation en sulfate d'ammonium (61.5g de sulfate d'ammonium pour 100ml d'échantillon). Le culot obtenu après centrifugation (25min., 10000rpm) est remis en solution dans du tampon Tris-HCl 50mM pH 8.3. Il est ensuite dialysé dans des membranes (Spectra/por, MWCO 3500Da) contre du tampon Tris-HCl 50 mM pH8.3

Le culot bactérien a été resuspendu dans du tampon Tris-HCl (Sigma) 50mM à pH 8,3. Les cellules ont ensuite été lysées à la presse de French, et le lysat obtenu a été ultracentrifugé à 27000g, pendant 2h45. Un demi-comprimé d'anti-protéase (Complete, EDTA-free, Roche) a été ajouté au surnageant obtenu.

L'extrait a ensuite été dialysé (Spectra/Por, MWCO 3500Da) contre du tampon Tris-HCl 50mM à pH 8,5, pendant une nuit, sous agitation à 4°C.

### Dosage du sulfate libéré

La production des activités sulfatases a été évaluée en mesurant la quantité de sulfate relarguée après incubation des différents carraghénanes en présence d'extraits bactériens. Les essais ont été conduits avec les surnageants de culture concentrés et les culots bactériens. Pour chaque échantillon analysé, un blanc est réalisé de manière similaire en ayant préalablement inactivé l'extrait enzymatique durant une quinzaine de minutes à 100°C.

Les milieux réactionnels ont été dilués par 2 avec de l'eau milliQ (Millipore) puis centrifugés en microcons (Amicon) avec un seuil de coupure de 10kDa. Cette centrifugation a été réalisée à 3300g pendant 90min à température ambiante. Le filtrat obtenu a ensuite été dosé par chromatographie échangeuse d'anions (HPAEC : High Performance Anion Exhange Chromatography) sur un système Dionex. 20µl d'échantillon ont été injectés grâce à un injecteur automatique (AS3000, Thermo). La séparation des anions présents dans les échantillons a été effectuée grâce à une colonne Ion-Pac AS11 (4x200mm, Dionex) munie d'une pré-colonne AG-11 (4x50mm, Dionex). Le système a été équilibré en NaOH 12mM. L'élution a été réalisée par un gradient isocratique en NaOH à un débit de 1ml/min (pompe GP40, Dionex). La détection des anions a été faite par conductimétrie avec un détecteur ED40 (Dionex) muni d'un suppresseur ASRS ultra-II-4mm (Dionex) fonctionnant à un courant de 198mA. Le logiciel utilisé pour l'acquisition et le traitement des données a été le logiciel Chroméléon 6.8. Grâce à une courbe étalon, l'aire des pics de sulfate a été convertie en parties par million (ppm). La différence entre la valeur de l'échantillon et celle du blanc a donné la quantité de sulfate libéré en ppm lors de la réaction enzymatique de désulfatation. Les résultats sont présentés dans le tableau 1 ci-dessous.

**Tableau 1**

| | | **Substrat** | | | | |
|---|---|---|---|---|---|---|
| **Extrait bactérien** | **Induction** | kappa | iota | lambda | kappa/mu | iota/nu |
| | kappa | 0,22 | 0 | 0,04 | 0,08 | 0,28 |
| Surnageant | iota | 1,36 | **39,36** | 1,26 | **21,96** | **18,96** |
| | lambda | 0,26 | 3,24 | 0,74 | 0,1 | 5,76 |
| | kappa | 0,04 | 3,4 | 0,08 | 0,4 | 4,28 |
| Culot | iota | 4,5 | **80,82** | 3,64 | 8,94 | **63,58** |
| | lambda | 0,36 | 12,9 | 0,04 | 1,08 | 17,3 |

Les résultats en gras correspondent aux conditions pour lesquelles les plus fortes activités sulfatases ont été observées.

### Purification et séquence protéique d'une nouvelle 4S-iota-carraghénane sulfatase

### Préparation de l'extrait bactérien

Le culot bactérien issu de la culture induite au iota-carraghénane et obtenu tel que précédemment décrit, contenant l'activité sulfatase, a été resuspendu dans du tampon Tris-HCl (Sigma) 50mM à pH 7,5. Les cellules ont ensuite été lysées à la presse de French, et le lysat obtenu a été ultracentrifugé à 27200g pendant 2h45. Un demi-comprimé d'anti-protéase (Complete, EDTA-free, Roche) a été ajouté au surnageant obtenu afin de limiter la dégradation des protéines.

Afin d'éliminer les molécules de petite taille (et notamment le sulfate libre) de l'extrait, celui-ci a été dialysé (Spectra/Por, MWCO 3500Da) contre du tampon Tris-HCl 50mM à pH 7,5 pendant une nuit, sous agitation à 4°C.

### Purification

Les expériences de purification ont été entreprises sur les sulfatases agissant sur le iota-carraghénane. Les étapes de purification ont été réalisées à l'aide d'un système Akta Purifier.

Le lysat a été déposé sur une colonne de chromatographie échangeuse d'anions faible DEAE Sepharose Fast Flow (GE Healthcare - 45 x 1 cm) préalablement équilibrée dans du tampon tris-HCl 50mM à pH 7,5. L'échantillon (environ 35ml) a été chargé à l'aide d'une superloop (ou boucle d'injection) à un débit de 2ml/min. La résine a ensuite été lavée avec ce même tampon jusqu'à obtenir une absorbance négligeable à 280nm. L'élution des protéines a été réalisée à un débit de 2ml/min par un gradient segmenté, croissant en NaCl de 0 à 1 M : 10 volumes de colonne de 0 à 500mM de NaCl et 2 volumes de colonne de 500mM à 1 M NaCl. Les fractions collectées d'un volume de 5,5ml ont été testées pour leur capacité à désulfater le iota-carraghénane.

Une fraction contenant un maximum d'activité sulfatase a ensuite été dialysée (membrane Spectra/Por, MWCO 3500Da) pendant 48h contre du tampon Tris-HCl 50mM à pH 7,5, sous agitation à 4°C. 1 ml de cette fraction a été déposé sur une résine échangeuse d'anions forte Q Fast Flow (GE Healthcare - Hitrap 1ml) préalablement équilibrée dans du tampon Tris-HCl 50mM à pH7,5. La résine a été lavée avec ce même tampon et les protéines ont été éluées avec un gradient croissant de NaCl de 0 à 1M réalisé de la manière suivante : 15 volumes de colonne de 0 à 500mM NaCl et 5 volumes de colonne de 500mM à 1M NaCl, à un débit de 1ml/min. Les fractions collectées, de 1ml ; ont été incubées en présence de iota-carraghénane pour mesurer l'activité sulfatase. Le degré de pureté des fractions a été visualisé par électrophorèse sur gel de polyacrylamide SDS-PAGE.

La pureté des fractions actives a été analysée par électrophorèse sur gel de polyacrylamide SDS-PAGE (Biorad, Criterion XT 12% Bis-Tris). A 15µl d'échantillon ont été ajoutés 5µl de tampon de charge 2% SDS (Amresco), 5% β-mercaptoéthanol (98% Sigma), 20% glycérol (CarloErba), 62,5mM Tris-HCl à pH 6,8 et 0,5% de bleu de Bromophénol (Sigma). Les échantillons ont ensuite été portés à ébullition pendant 3min afin de dénaturer les protéines. Les 20µl de mélange ont alors été déposés sur le gel. Les dépôts de 5µl de marqueurs de taille (Biorad, *Precision Plus Protein)* ont permis d'évaluer le poids moléculaire des protéines comprises entre 10 et 250kDa. La migration a été faite à température ambiante, à 110volts (pour 1 gel) durant 2h, dans un tampon de migration composé de MOPS (Sigma) 200mM, Tris 250mM pH 8,1 et SDS 5g/l. La révélation du gel a été réalisée à l'aide d'une coloration au bleu de Coomassie colloïdal (Candiano et al., Electrophoresis, 25(9) : 1327-1333, 2004) [5]. Le poids moléculaire de la protéine purifiée a été estimé à environ 55 kDa (figure 4).

### Séquence protéique et nucléique

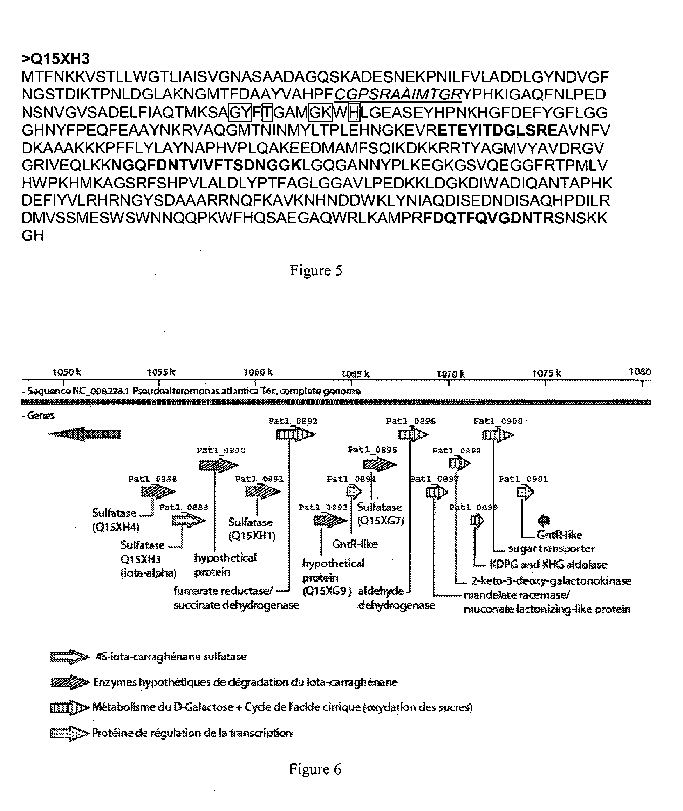

La bande de la protéine a été excisée du gel, digérée à la trypsine et les peptides obtenus ont été séquencés par spectrométrie de masse sur la plate-forme RIO « Biopolymères » localisée à l'INRA de Nantes. Les trois séquences déterminées [NGQFDNTVIVFTSDNGGK (SEQ ID NO: 1), FDQTFQVGDNTR (SEQ ID NO : 2), et ETEYITDGLSR (SEQ ID NO : 3)] ont été confrontées à la banque TrEMBL. Les trois peptides correspondent à 100% à la protéine Q15XH3 (figure 5, SEQ ID NO: 4) de *Pseudoalteromonas atlantica* T6c dont le gène (Patl_0889) a été annoté comme une sulfatase (Copeland et al., 2006, précité) [4]. La protéine présente la séquence consensus de 12 acides aminés (C/S-X-P-S/X-R-XXX-L/X-G/X-R/X, SEQ ID NO : 5) requise pour la conversion de la cystéine en formylglycine (FGly) et les acides aminés catalytiques présents dans la séquence conservée (G-Y/V-X-S/T-XXX-G-K-X-X-H, SEQ ID NO : 6). L'environnement génomique du gène de la nouvelle sulfatase converge vers une implication de cette protéine dans la dégradation du iota-carraghénane (figure 6). En effet, le gène de Q15XH3 est localisé dans un cluster de gènes qui contient d'autres sulfatases (dont Q15XG7 qui possède 41% d'identité avec Q15XH3), deux protéines inconnues, mais surtout, plusieurs gènes du cycle citrique (oxydation des sucres) et de métabolisme du D-galactose sont clairement identifiés. Les fonctions des sulfatases présentes dans ce cluster sont probablement liées à la désulfatation du iota- et/ou alpha-carraghénane.

### Activité sulfatase

Afin de mesurer l'activité sulfatase, 100µl d'échantillon à doser ont été mis en présence de 100µl d'une solution de iota-carraghénane (CP-Kelco n°1256) à 1% dans du tampon Tris-HCl 50mM à pH 7,5. Les réactions enzymatiques ont été réalisées à 34°C en bain-marie durant 48h. Pour chaque échantillon, un blanc a été effectué dans des conditions similaires en ayant préalablement inactivé l'extrait enzymatique durant 15min, à 100°C.

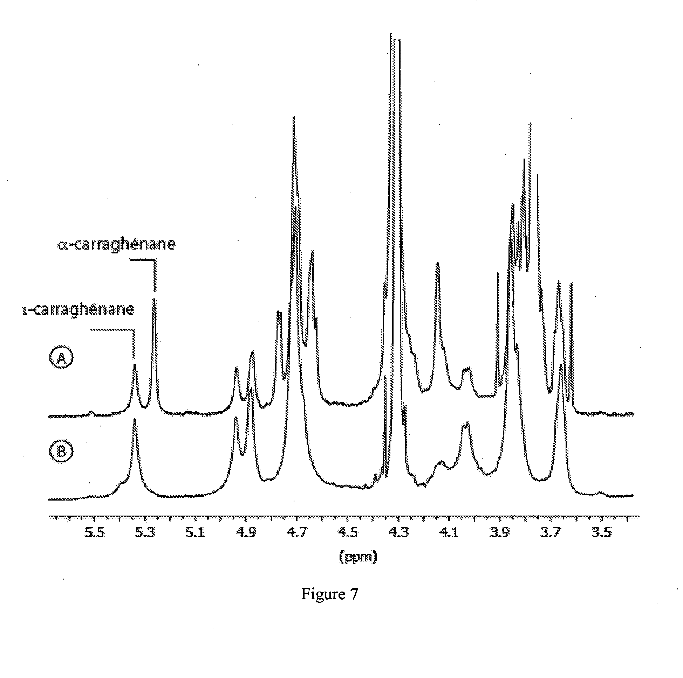

La position du groupement sulfaté clivé et donc l'identification du produit formé lors de l'hydrolyse enzymatique, a été réalisée par RMN. Pour cette analyse, les réactions de désulfatation ont été faites en incubant 700µl de iota-carraghénane à 1% (CP-Kelco n°1256) en présence de 300µl d'extrait bactérien. Les mélanges réactionnels ont été incubés à 34°C en bain-marie durant 72h puis lyophilisés. Les échantillons ont alors été échangés deux fois dans du D₂O puis redissous dans 700µl de D₂O à 99,97% pour être à une concentration approximative de 10mg/ml. Les spectres ¹H-RMN ont été enregistrés à 70°C sur un spectrophotomètre BRUKER Avance DRX 500 par le service RMN (Université de Bretagne Occidentale, Brest). Les protons anomériques des carraghénanes présentent des déplacements chimiques (d) caractéristiques compris entre 5 et 5,6 ppm environ. Les résultats montrent que la désulfatation du iota-carraghénane par la protéine pure a conduit à la production d'alpha-carraghénane (figure 7A), comme dans le cas de la première 4S-iota-carraghénane sulfatase purifiée mais dont le poids moléculaire était de 115 kDa (Demande de Brevet français FR 09/52642, précité) [3].

Une nouvelle sulfatase capable de convertir du iota- en alpha-carraghénane a donc été identifiée. Elle se distingue de l'unique 4S-iota-carraghénane sulfatase connue à ce jour qui appartient à la famille des amidohydrolases.

La séquence de la nouvelle carraghénane sulfatase (Q15XH3) montre qu'elle appartient à la famille des FGly-sulfatases. Cette FGly-sulfatase est la première carraghénane sulfatase de cette famille qui contient d'autres enzymes agissant sur des carbohydrates. Des sulfatases agissant sur les glycosaminoglycanes (i.e. héparine) et des cérébrosides ont été décrites.

### Listes des références

1. Bixler et al., Food Hydocolloids, 15 : 619-630, 2001
2. Bixler and Porse, J. Appl. Phycol., 2010, online
3. Demande de Brevet français FR 09/52642, publiée sous le numéro FR 2944804
4. Copeland et al., « Complete sequence of *Pseudoalteromonas atlantica* T6c", EMBL ACCESSION N° CP000388, PROTEIN_ID ABG39415.1, 2006
5. Candiano et al., Electrophoresis, 25(9) : 1327-1333, 2004

## Revendications

1. Procédé de transformation du iota-carraghénane en alpha-carraghénane comprenant la catalyse enzymatique de la conversion du motif iota-carrabiose en motif alpha-carrabiose par une enzyme ayant une activité sulfatase et dont la séquence peptidique comprend les peptides de séquences suivantes : SEQ ID NO 1, SEQ ID NO 2, et SEQ ID NO 3.

2. Procédé selon la revendication 1 dans lequel la séquence peptidique de l'enzyme est la séquence SEQ ID NO 4.

3. Procédé selon l'une quelconque des revendications 1 à 2, dans lequel ladite enzyme est une 4S-iota-carraghénane sulfatase.

4. Procédé selon l'une quelconque des revendications 1 à 3, dans lequel l'enzyme est produite par une cellule hôte comprenant un acide nucléique codant pour ladite enzyme et/ou un vecteur comprenant une séquence d'acide nucléique codant pour ladite enzyme.

## Patentansprüche

1. Verfahren zur Umwandlung von Iota-Carrageen in Alpha-Carrageen, wobei das Verfahren die enzymatische Katalyse der Konvertierung des Iota-Carrabiose-Motivs in ein Alpha-Carrabiose-Motiv durch ein Enzym umfasst, das eine Sulfatase-Aktivität aufweist und dessen Peptidsequenz die Peptide mit den folgenden Sequenzen umfasst: SEQ ID Nr. 1, SEQ ID Nr. 2 und SEQ ID Nr. 3.

2. Verfahren nach Anspruch 1, wobei die Peptidsequenz des Enzyms die Sequenz SEQ ID Nr. 4 ist.

3. Verfahren nach einem der Ansprüche 1 bis 2, wobei das besagte Enzym eine 4S-Iota-Carrageen-Sulfatase ist.

4. Verfahren nach einem der Ansprüche 1 bis 3, wobei das Enzym durch eine Wirtszelle produziert wird, die eine Nukleinsäure umfasst, die für das besagte Enzym kodiert, und/oder einen Vektor, der eine Nukleinsäuresequenz umfasst, die für das besagte Enzym kodiert.

## Claims

1. A method for transforming iota-carrageenan to alpha-carrageenan comprising the enzymatic catalysis of the conversion of the iota-carrabiose motif to the alpha-carrabiose motif by an enzyme having a sulfatase activity and which peptide sequence comprises the peptides having the following sequences: SEQ ID NO:1, SEQ ID NO:2, and SEQ ID NO:3.

2. The method as claimed in claim 1, in which the peptide sequence of the enzyme is the sequence SEQ ID NO:4.

3. The method as claimed in either of claims 1 and 2, in which said enzyme is a 4S-iota-carrageenan sulfatase.

4. The method as claimed in any one of claims 1 to 3, in which the enzyme is produced by a host cell comprising a nucleic acid encoding said enzyme and/or a vector comprising a nucleic acid sequence encoding said enzyme.
